# EUROPEAN PATENT APPLICATION

(11) **EP 4 745 571 A1**
(43) Date of publication of application: **20.05.2026**
(21) Application number: 24839740.8
(22) Date of filing: 09.07.2024
(51) Int. Cl.: G01N 33/50, G01N 27/62

(54) **METHOD FOR ASSESSING DISORDER OF MUCOUS MEMBRANE OF GASTROINTESTINAL TRACT**

(30) Priority: 12.07.2023 JP 2023114423
(71) Applicant: SHIMADZU CORPORATION, Kyoto-shi, Kyoto 604-8511 (JP); Hyogo Medical University, Nishinomiya-shi, Hyogo 663-8501 (JP)
(72) Inventor: YOKOYAMA, Tomonori, Kyoto-shi, Kyoto 604-8511 (JP); OJIMA, Noriyuki, Kyoto-shi, Kyoto 604-8511 (JP); KIKUCHI, Shojiro, Nishinomiya-shi, Hyogo 663-8501 (JP); FUKUI, Hirokazu, Nishinomiya-shi, Hyogo 663-8501 (JP)
(74) Representative: Müller-Boré & Partner Patentanwälte PartG mbB
(86) International application number: PCT/JP2024/024733
(87) International publication number: WO 2025/013850

(57) **Abstract**

A method for determining a mucosal disorder in a gastrointestinal tract includes: a preparation step for preparing a biological sample collected from a subject to which an endoscopic dye compound has been administered; a measurement step for measuring an amount of the endoscopic dye compound or a metabolite thereof in the biological sample; a comparison step for comparing the measured amount of the endoscopic dye compound or a metabolite thereof with a reference value; and a determination step for determining that the subject is highly likely to have a mucosal disorder in the gastrointestinal tract if the measured amount of the endoscopic dye compound or a metabolite thereof is equal to or greater than the reference value.

## Description

### TECHNICAL FIELD

The present invention relates to a method for determining a mucosal disorder in a gastrointestinal tract.

### BACKGROUND ART

The gastrointestinal tract (such as esophagus, stomach, small intestine, and large intestine) prevents pathogens or allergic substances from entering the body due to the barrier function of mucosal cells and the activity of immune cells. When the barrier function of the mucosal cells declines, pathogens, allergic substances or the like can easily enter the body. This symptom is called "hyperpermeability of gastrointestinal mucosa".

Hyperpermeability of gastrointestinal mucosa occurs in various diseases such as inflammatory bowel diseases, functional gastrointestinal disorders, cancers, and lifestyle-related diseases. An objective evaluation of the permeability of the gastrointestinal mucosa is useful for determining the activity or therapeutic effect of various diseases associated with mucosal disorders. Currently established methods for objectively evaluating the permeability of the gastrointestinal mucosa and the degree of a mucosal disorder include administering sugars (such as lactulose and mannitol) with different absorption rates and measuring the concentration of each sugar excreted to the urine (Eur. Rev. Med. Pharmacol. Sci., 2019, 23, 795-810 (NPL 1)).

### CITATION LIST

### NON-PATENT LITERATURE

NPL 1: Eur. Rev. Med. Pharmacol. Sci., 2019, 23, 795-810.

### SUMMARY OF INVENTION

### TECHNICAL PROBLEM

The evaluation method described in NPL 1 requires hospitalization, long-term fasting, or collection of a day's worth of urine, which is burdensome to patients. In addition, the evaluation method described in NPL 1 tends to yield unstable evaluation results.

The present invention has been made in view of the above-mentioned problems, and an object of the present invention is to provide a method for determining a mucosal disorder in a gastrointestinal tract, which is simple and capable of evaluating the degree of a mucosa disorder.

### SOLUTION TO PROBLEM

As a result of intensive research, the inventors of the present invention have found that the degree of a mucosal disorder can be evaluated by measuring an amount of an endoscopic dye compound or a metabolite thereof in a biological sample collected from a subject to which the endoscopic dye compound has been administered, and thereby have completed the present invention.

A first aspect of the present invention relates to a method for determining a mucosal disorder in a gastrointestinal tract, and the determination method includes:
a preparation step for preparing a biological sample collected from a subject to which an endoscopic dye compound has been administered;
a measurement step for measuring an amount of the endoscopic dye compound or a metabolite thereof in the biological sample;
a comparison step for comparing the measured amount of the endoscopic dye compound or a metabolite thereof with a reference value; and
a determination step for determining that the subject is highly likely to have a mucosal disorder in the gastrointestinal tract if the measured amount of the endoscopic dye compound or a metabolite thereof is equal to or greater than the reference value.

### ADVANTAGEOUS EFFECTS OF INVENTION

According to the present invention, it is possible to provide a method for determining a mucosal disorder in a gastrointestinal tract, which is simple and capable of evaluating the degree of a mucosa disorder.

### BRIEF DESCRIPTION OF DRAWINGS

Fig. 1 is a graph illustrating concentrations of indigo carmine in blood for a control group (subject A).
Fig. 2 is a graph illustrating concentrations of indigo carmine in blood for a mucosal disorder group (subject B).

### DESCRIPTION OF EMBODIMENTS

Hereinafter, an embodiment of the present invention (will be referred to as the present embodiment below) will be described. However, the present embodiment is not limited thereto. In the present specification, the expression in the form of "A to Z" denotes an upper limit and a lower limit of a range (in other words, A or more and Z or less), and if no unit is defined for A but a unit is defined for Z, the unit of A is the same as the unit of Z.

### Method for Determining Mucosal Disorder in Gastrointestinal Tract

The determination method according to the present embodiment is a method for determining a mucosal disorder in a gastrointestinal tract, and the determination method includes:
a preparation step for preparing a biological sample collected from a subject to which an endoscopic dye compound has been administered;
a measurement step for measuring an amount of the endoscopic dye compound or a metabolite thereof in the biological sample;
a comparison step for comparing the measured amount of the endoscopic dye compound or a metabolite thereof with a reference value; and
a determination step for determining that the subject is highly likely to have a mucosal disorder in the gastrointestinal tract if the measured amount of the endoscopic dye compound or a metabolite thereof is equal to or greater than the reference value.

### <Preparation Step>

In this step, a biological sample collected from a subject to which an endoscopic dye compound has been administered is prepared. In the present embodiment, "the endoscopic dye compound" refers to such a dye compound that is used for endoscopy and satisfies the following (A) and (B):
(A) Dyes, pigments, or compounds made by synthetic processes or similar artificial techniques, or compounds extracted, isolated or derived from plants, animals, minerals or other sources with or without intermediate or final identity changes; and
(B) Compounds that can impart color (including black, white, or gray color) when added to or applied to foods, pharmaceuticals, cosmetics, or the human body or any part thereof.

Examples of the endoscopic dye compound include compounds described in the list of "Dyes used for endoscopy" for United States Food and Drug Administration (FDA) (https://www.ecfr.gov/current/title-21/chapter-1/subchapter-A/part-74). In the present embodiment, the endoscopic dye compound preferably contains at least one selected from the group consisting of phthalocyanine blue, D&C Black No. 4, FD&C Blue No. 2 (indigo carmine), D&C Blue No.6, D&C Green No.6, D&C Red No.17, D&C Violet No.2, D&C Yellow No. 8, and D&C Yellow No. 10. In one aspect of the present embodiment, the endoscopic dye compound preferably contains indigo carmine. The IUPAC name for indigo carmine is disodium 3,3'-dioxo-2,2'-bis-indolidene-5,5'-disulfonate.

In one aspect of the present embodiment, the endoscopic dye compound is preferably a poorly absorbable compound. The abovementioned indigo carmine is a poorly absorbable compound. In the present embodiment, the "poorly absorbable compound" refers to a compound that exhibits a low absorption rate from the mucosa of a gastrointestinal tract when administered to a healthy subject without a mucosal disorder in the gastrointestinal tract. The poorly absorbable compound may be, for example, a compound which has been clinically used and has established safety.

In the present embodiment, the term "subject" is not particularly limited as long as it is a mammal to which an endoscopic dye compound is administered, and examples thereof include humans, chimpanzees, cows, sheep, pigs, goats, horses, dogs, cats, mice, rats, rabbits, and guinea pigs. In one aspect of the present embodiment, the subject is preferably a human. The term "subject" may also include adults, infants, and newborns.

The method of administering an endoscopic dye compound to the subject may be any known method without any particular limitation. In one aspect of the present embodiment, the method of administering an endoscopic dye compound to the subject may be a method of directly spraying the dye compound to a target site of the gastrointestinal mucosa or may be an oral administration method. In the present embodiment, the term "target site" refers to a site to be evaluated in the gastrointestinal mucosa. Examples of the target site include a lesion site.

When the endoscopic dye compound is administered by oral administration, the dosage form of a medical agent that contains the endoscopic dye compound is not particularly limited as long as it can reach the target site of the gastrointestinal mucosa, and examples thereof include tablets, capsules, and powders.

The dosage of the endoscopic dye compound may vary depending on the body weight, age, and the like of the subject, and may be, for example, a clinical dosage for endoscopy (for example, 20 mg/20 mL in the case of indigo carmine).

In the present embodiment, the term "biological sample" refers to such a biological sample that may contain an endoscopic dye compound administered to a subject or a metabolite thereof and is collected from the subject. Examples of the biological sample include blood, plasma, serum, and urine. In one aspect of the present embodiment, the biological sample is preferably blood, serum, plasma, or urine.

The method of collecting the biological sample is not particularly limited and may be any known method. For example, blood may be collected by blood collection using a syringe or the like.

In one aspect of the present embodiment, the biological sample is preferably a biological sample collected from a subject to which an endoscopic dye compound has been administered by directly spraying the endoscopic dye compound to a target site of the gastrointestinal mucosa. Example methods of directly spraying the endoscopic dye compound to the target site of the gastrointestinal mucosa include a method of spraying the endoscopic dye compound using an endoscope. By administering the endoscopic dye compound in this manner, it is possible to determine the presence or absence of a mucosal disorder and the degree of the mucosal disorder for each target site.

The time of collecting the biological sample may be any time in a period between a time "after the endoscopic dye compound or a metabolite thereof has been transferred to the biological sample" to a time "before the endoscopic dye compound or a metabolite thereof has been released from the biological sample" via the blood stream, without any particular limitation. In one aspect of the present embodiment, the time of collecting the sample is preferably any time from 30 minutes to 120 minutes after the endoscopic dye compound has been administered to the subject, and more preferably any time from 30 minutes to 60 minutes after the endoscopic dye compound has been administered to the subject. In one aspect of the present embodiment, the number of times of collecting the biological sample may be one or may be a plurality of times (for example, 2 to 5 times) by changing the time of collecting the biological sample. When the biological sample is collected a plurality of times at different times, it is possible to investigate changes over time in the amount of the endoscopic dye compound or a metabolite thereof in the biological sample.

In one aspect of the present embodiment, a biological sample collected from a subject may be pretreated by a method well known to a person skilled in the art prior to the measurement step to be described below. For example, when the biological sample is blood, peripheral blood of a subject is firstly collected into a blood collection tube, coagulated, and then centrifuged to obtain a supernatant. The obtained supernatant fraction may be subjected to a pretreatment such as solid phase extraction to obtain a measurement sample.

### <Measurement Step>

In this step, the amount of the endoscopic dye compound or a metabolite thereof in the biological sample is measured. In the present embodiment, a metabolite of the endoscopy dye compound refers to a compound produced by converting the endoscopy dye compound according to the biological functions of the administered subject. For example, the metabolite of indigo carmine may be 2-amino-5-sulfobenzoic acid (5-sulfoanthranilic acid), 2,3-dihydro-2,3-dioxo-1H-indole-5-sulfonic acid (isatin-5-sulfonic acid), or the like. In other words, the metabolite of indigo carmine is preferably isatin-5-sulfonic acid or 5-sulfoanthranilic acid.

In the present embodiment, the method for measuring the amount of the endoscopic dye compound or a metabolite thereof is not particularly limited, and examples thereof include ELISA (Enzyme-Linked Immunosorbent Assay), mass spectrometry, liquid chromatography, immunochromatography, fluorometric detection method, spectrophotometry, and the like.

Examples of a column for liquid chromatography include a reverse phase column, a HILIC (Hydrophilic Interaction Liquid Chromatography) column, and the like.

The mobile phase for liquid chromatography may be appropriately selected depending on the column to be used. For example, when a reverse phase column is used, methanol, acetonitrile, an ammonium acetate solution, or the like may be preferably used.

Examples of mass spectrometry include liquid chromatography mass spectrometry (LC-MS), laser desorption/ionization mass spectrometry (LDI-MS), and the like. The equipment to be used in mass spectrometry is not particularly limited as long as it is commercially available, and examples thereof include LCMS-8060 and AXIMA Performance manufactured by Shimadzu Corporation.

In one aspect of the present embodiment, in the measurement step, it is preferable to measure the amount of the endoscopic dye compound or a metabolite thereof using LC-MS or LDI-MS.

In the present embodiment, the "amount of an endoscopic dye compound or a metabolite thereof" is not particularly limited as long as it is a physical amount indicating the presence of the endoscopic dye compound or a metabolite thereof in a biological sample, and examples thereof include a density of the endoscopic dye compound or a metabolite thereof in the biological sample and a concentration of the endoscopic dye compound or a metabolite thereof in the biological sample. When the biological sample is blood, serum, or the like, the "amount of an endoscopic dye compound or a metabolite thereof" is generally expressed as blood concentration.

In one aspect of the present embodiment, the measurement may be performed in the presence of an internal standard material where appropriate. Examples of the internal standard material include a tar dye such as orange G and sunset yellow FCF.

### <Comparison Step>

In this step, the measured amount of the endoscopic dye compound or a metabolite thereof is compared with a reference value.

In the present embodiment, the "reference value" is an "amount of an endoscopic dye compound or a metabolite thereof" which serves as a reference for determining whether the subject is highly likely to have a mucosal disorder in the gastrointestinal tract. The reference value may be arbitrarily set. For example, the reference value may be an "amount of an endoscopic dye compound or a metabolite thereof" in a subject (such as a healthy subject) without a mucosal disorder in the gastrointestinal tract, or may be a preset amount. The preset amount may be appropriately set as a value that is not included in the measured values for the patient group among the healthy individuals (healthy group) and the patients (patient group) having a mucosal disorder in the gastrointestinal tract, or a value that is not for either the healthy group or the patient group but lies between the value for the healthy group and the value for the patient group.

### <Determination Step>

In this step, when the measured amount of the endoscopic dye compound or a metabolite thereof is equal to or greater than the reference value, it is determined that the subject is highly likely to have a mucosal disorder in the gastrointestinal tract.

In one aspect of the present embodiment, the expression "when the measured amount of the endoscopic dye compound or a metabolite thereof is equal to or greater than the reference value" is a concept that includes a case where the measured amount of the endoscopy dye compound or a metabolite thereof is equal to the reference value and a case where the measured amount of the endoscopy dye compound or a metabolite thereof is greater than the reference value.

In another aspect of the present embodiment, the greater the measured amount of the endoscopic dye compound or a metabolite thereof is, the greater the degree of a mucosal disorder in the gastrointestinal tract may be.

In one aspect of the present embodiment, the mucosal disorder in the gastrointestinal tract preferably includes irritable bowel syndrome (IBS) and inflammatory bowel disease (IBD). Examples of the inflammatory bowel disease include ulcerative colitis.

Conventionally, the presence or absence of a mucosal disorder has been evaluated by spraying an endoscopic dye compound such as indigo carmine on the inner wall of the large intestine and observing the dye compound with an endoscope (contrast method). When a site has a mucosal disorder, the surface of the site becomes rough (uneven), which makes it easy for indigo carmine to accumulate, resulting in a dark blue color in the site. However, the contrast method was not able to quantitatively evaluate the degree of the mucosal disorder. The determination method according to the present embodiment focuses on the fact that the endoscopic dye compound such as indigo carmine is more likely to be absorbed in a site with a mucosal disorder than in the normal mucosa, and quantitatively evaluates the permeability of the endoscopic dye compound such as indigo carmine by measuring the blood concentration thereof. Therefore, the determination method according to the present embodiment can quantitatively evaluate the degree of a mucosal disorder.

In addition, according to the determination method of the present embodiment, by quantitatively evaluating a mucosal disorder in the gastrointestinal tract at a target site sprayed with an endoscopic dye compound, it is possible to evaluate the pathological condition of a disease having a symptom of an intestinal mucosal disorder such as irritable bowel syndrome (IBS) and inflammatory bowel disease (IBD). It is also expected that the determination method of the present embodiment may be applied to "a disease having a symptom of a gastrointestinal mucosal disorder" such as cancer, metabolic disease, diabetes, and neuropsychiatric disease, may be used to confirm the effect of a therapeutic agent, and may be used in a therapeutic plan such as changing or adjusting a therapeutic agent according to the clinical conditions of a patient.

### [Examples]

Hereinafter, the present invention will be described in more detail with reference to the examples, but the present invention is not limited thereto.

Indigo carmine (which is a poorly absorbable compound) was administered to subjects (targets) in a control group and a mucosal disorder group shown below, and the amount (concentration) of indigo carmine in the blood was evaluated. The specific procedure is shown below. Subjects having an age of 20 years or older and having no surgical history of the gastrointestinal tract were selected for both the control group and the mucosal disorder group.

Control group: subjects without severe diarrhea or constipation and without colonic inflammation, ulcers, cancer, other diseases confirmed by endoscopy (Mayo classification: Grade 0).

Mucosal Disorder Group: subjects clinically diagnosed with inflammatory bowel disease (IBD) (Mayo classification: Grade 2 or 3).

### <Preparation Step>

For subject A in the control group and each of subjects B to D in the mucosal disorder group, indigo carmine was sprayed to an upper portion of the large intestine (cecum) using an endoscope. Blood was collected immediately before spraying, 30 minutes after spraying, and 60 minutes after spraying. The collected blood was allowed to stand to coagulate blood cell components without adding an anticoagulant. Thereafter, the blood was separated into serum and blood clot by centrifugation, and the serum was collected as a measurement sample. The serum corresponds to "a biological sample collected from a subject to which an endoscopic dye compound has been administered".

A methanol solution (100 µl) that contains Orange G (manufactured by FUJIFILM Wako Pure Chemical Corporation and having a final concentration of 100 ng/ml) was added to the serum (20 µl) to obtain a mixture. The obtained mixture was centrifuged (12,000×g, 10 minutes), and the supernatant (80 µl) was collected. To the collected supernatant, 5% methanol formic acid (20 µl) was added to obtain a mixture. The mixture was purified by passing the mixture through a pre-conditioned affinity column (MonoSpin Phospholipid, manufactured by GL Sciences), and the filtrate was collected. The collected filtrate was vacuum dried and then redissolved in ultrapure water (150 µl) to obtain an analytical sample.

### <Measurement Step>

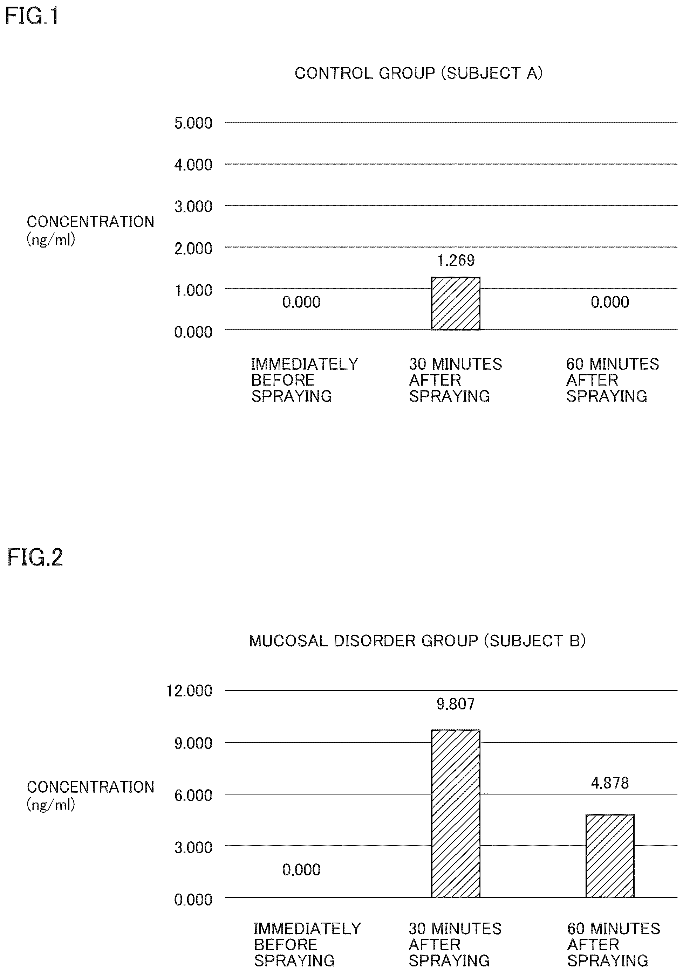

The analytical sample was analyzed using a high-speed liquid chromatography/mass spectrometer (LC-MS/MS) (product name: LCMS-8060 manufactured by Shimadzu Corporation) under the following conditions (Table 1). The amount of indigo carmine was calculated from the obtained analysis results according to the following method. The results are shown in Fig. 1 (control group: subject A) and Fig. 2 (mucosal disorder group: subject B) as well as Table 2.

### (LC-MS/MS Analytical Conditions)

<LC Conditions>
Column: Shim-pack XR-ODS II (3.9 mm I.D. × 75 mm, 2.2 µm)
Column oven: 40°C
Mobile phase:
   A: 10 mM ammonium acetate solution; and
   B: acetonitrile
Gradient: 3% (0 min)→3% (0.25 min)→95% (5 min)→95% (7 min)-3% (7.1 min)→3% (10 min)
Flow rate: 0.4 ml/min
Sample cooler: 4°C
Injection volume: 5 µl

### <MS Conditions>

| Compound | Ch. | Precursor (m/z) | Product (m/z) | Dwell Time (msec) | Q1 Pre Bias (V) | CE (V) | Q3 Pre Bias (V) | Resolution Power | Measurement Time (min) |
|---|---|---|---|---|---|---|---|---|---|
| Indigo Carmine | Ch.1 | -421.00 | 3-1-1.00 | 100 | 12.0 | 31.0 | 25.0 | Unit - Unit | 1.5 - 3.0 |
| | Ch.2 | -421.00 | 261.00 | 100 | 12.0 | 40.0 | 27.0 | | |
| Orange G | Ch.1 | -1-07.00 | 302.00 | 10 | 11.0 | 19.0 | 15.0 | Unit - Unit | 1.5 - 3.0 |
| | Ch.2 | -1-07.00 | 207.00 | 10 | 26.0 | 33,0 | 20.0 | | |

Nebulizer gas flow rate: 3 L/min
Heating gas flow rate: 10 L/min
Interface temperature: 300°C
Solvent removal temperature: 526°C
DL temperature: 250°C
Heat block temperature: 400°C
Drying gas flow rate: 10 L/min

**[Table 2]**

| | | Mayo Classification | Blood Concentration of Indigo Carmine (ng/ml) | | |
|---|---|---|---|---|---|
| | | | Immediately Before Spraying | 30 Minutes After Spraying | 60 Minutes After Spraying |
| Control Group | Subject A | Grade 0 Normal | 0.000 | 1.269 | 0.000 |
| Mucosal Disorder Group | Subject B | Grade 3 Severe | 0.000 | 9.807 | 4.878 |
| | Subject C | Grade 2 Moderate | 0.000 | 2.781 | 1.741 |
| | Subject D | Grade 2 Moderate | 0.000 | 3.857 | 1.192 |

### <Comparison Step & Determination Step>

From the results of Fig. 1 and Table 2, in the control group (subject A), the presence of indigo carmine in the blood was confirmed 30 minutes after spraying (1.269 ng/ml), and the presence of indigo carmine was not confirmed 60 minutes after spraying. On the other hand, from the results of Fig. 2 and Table 2, in the mucosal disorder group (subjects B to D), the presence of indigo carmine was confirmed at a high concentration in the blood 30 minutes after spraying (2.781 to 9.807 ng/ml), and the presence of indigo carmine was also confirmed 60 minutes after spraying (1.192 to 4.878 ng/ml). Furthermore, in the mucosal disorder group, the blood concentration of indigo carmine increased as the grade of Mayo classification progressed, and a correlation was observed therebetween.

From the above results, it was found that the concentration of indigo carmine in the blood of the mucosal disorder group was higher than that of the control group. It is considered that the reason therefor is because the absorption of indigo carmine from the gastrointestinal tract increases due to the mucosal disorder of the gastrointestinal tract. It was also suggested that the degree of the mucosal disorder can be quantitatively evaluated by measuring the concentration of indigo carmine in the blood after endoscopy. Since indigo carmine in the blood will be excreted to urine, it is expected that the same result will be obtained when urine is used as the biological sample.

### [Aspects]

It will be understood by a person skilled in the art that the embodiments and examples described above are specific examples of the following aspects.

### (First Aspect)

A determination method according to an embodiment is a method for determining a mucosal disorder in a gastrointestinal tract, and the method includes: a preparation step for preparing a biological sample collected from a subject to which an endoscopic dye compound has been administered; a measurement step for measuring an amount of the endoscopic dye compound or a metabolite thereof in the biological sample; a comparison step for comparing the measured amount of the endoscopic dye compound or a metabolite thereof with a reference value; and a determination step for determining that the subject is highly likely to have a mucosal disorder in the gastrointestinal tract if the measured amount of the endoscopic dye compound or a metabolite thereof is equal to or greater than the reference value. According to the determination method described in the first aspect, it is possible to provide a method for determining a mucosal disorder in a gastrointestinal tract, which is simple and capable of evaluating the degree of a mucosa disorder.

### (Second Aspect)

In the determination method described in the first aspect, the endoscopy dye compound includes at least one selected from the group consisting of phthalocyanine blue, D&C Black No. 4, indigo carmine, D&C Blue No.6, D&C Green No.6, D&C Red No.17, D&C Violet No.2, D&C Yellow No. 8, and D&C Yellow No. 10. According to the determination method described in the second aspect, it is possible to determine a mucosal disorder in the gastrointestinal tract more easily.

### (Third Aspect)

In the determination method according to the first aspect or the second aspect, the biological sample is blood, serum, plasma, or urine. According to the determination method described in the third aspect, it is possible to determine a mucosal disorder in the gastrointestinal tract more easily.

### (Fourth Aspect)

In the determination method according to any one of the first to third aspects, in the measurement step, the amount of the endoscopic dye compound or a metabolite thereof is measured using LC-MS or LDI-MS. According to the determination method described in the fourth aspect, it is possible to evaluate the degree of a mucosal disorder more quantitatively.

### (Fifth Aspect)

In the determination method according to any one of the first to fourth aspects, the biological sample is collected from a subject to which the endoscopic dye compound has been administered by directly spraying the endoscopic dye compound to a target site of the gastrointestinal mucosa. According to the determination method described in the fifth aspect, it is possible to evaluate the degree of a mucosal disorder for each target site.

### (Sixth Aspect)

In the determination method according to any one of the first to fifth aspects, the mucosal disorder in the gastrointestinal tract includes irritable bowel syndrome and inflammatory bowel disease. According to the determination method described in the sixth aspect, it is possible to suitably evaluate the degree of irritable bowel syndrome or inflammatory bowel disease.

## Claims

1. A method for determining a mucosal disorder in a gastrointestinal tract, the determination method comprising:
a preparation step for preparing a biological sample collected from a subject to which an endoscopic dye compound has been administered;
a measurement step for measuring an amount of the endoscopic dye compound or a metabolite thereof in the biological sample;
a comparison step for comparing the measured amount of the endoscopic dye compound or a metabolite thereof with a reference value; and
a determination step for determining that the subject is highly likely to have a mucosal disorder in the gastrointestinal tract if the measured amount of the endoscopic dye compound or a metabolite thereof is equal to or greater than the reference value.

2. The determination method according to claim 1, wherein
the endoscopic dye compound includes at least one selected from the group consisting of phthalocyanine blue, D&C Black No. 4, indigo carmine, D&C Blue No.6, D&C Green No.6, D&C Red No.17, D&C Violet No.2, D&C Yellow No. 8, and D&C Yellow No. 10.

3. The determination method according to claim 1 or claim 2, wherein
the biological sample is blood, serum, plasma, or urine.

4. The determination method according to claim 1 or claim 2, wherein
in the measurement step, the amount of the endoscopic dye compound or a metabolite thereof is measured using LC-MS or LDI-MS.

5. The determination method according to claim 1 or claim 2, wherein
the biological sample is collected from a subject to which the endoscopic dye compound has been administered by directly spraying the endoscopic dye compound to a target site of a gastrointestinal mucosa.

6. The determination method according to claim 1 or claim 2, wherein
the mucosal disorder in the gastrointestinal tract includes irritable bowel syndrome and inflammatory bowel disease.
